Europäisches Patentamt

European Patent Office

Office européen des brevets

(11) Numéro de publication : **0 479 781 B1**

# FASCICULE DE BREVET EUROPEEN

(45) Date de publication du fascicule du brevet :
**22.09.93 Bulletin 93/38**

(51) Int. Cl.$^5$ : **A61K 9/127, A61K 31/71**

(21) Numéro de dépôt : **89911009.2**

(22) Date de dépôt : **12.10.89**

(86) Numéro de dépôt international :
**PCT/BE89/00047**

(87) Numéro de publication internationale :
**WO 90/03781 19.04.90 Gazette 90/09**

(54) **COMPOSITION PHARMACEUTIQUE CONTENANT UN COMPOSE ACTIF HYDROPHILE, TRAITE PAR UN ACIDE ORGANIQUE ET ENCAPSULE DANS UN LIPOSOME.**

(30) Priorité : **12.10.88 BE 8801180**

(43) Date de publication de la demande :
**15.04.92 Bulletin 92/16**

(45) Mention de la délivrance du brevet :
**22.09.93 Bulletin 93/38**

(84) Etats contractants désignés :
**AT BE CH DE FR GB IT LI LU NL SE**

(56) Documents cités :
**EP-A- 211 647**
**WO-A-87/00043**
**WO-A-88/04573**
**JOURNAL OF CONTROLLED RELEASE, vol. 5, no. 2, September 1987, Elsevier Science Publishers BV, Amsterdam (NL); H.SCHREIER et al., pp. 187-192**
**CHEMICAL ABSTRACTS, vol. 101, no. 6, 06 August 1984, Columbus, OH (US); M.BARZA et al., p. 3, no. 43477g**

(73) Titulaire : **LEGROS, Franz**
**8, rue de Plagniau**
**B-133o Rixensart (BE)**
Titulaire : **LEROY, Claude-Pascal**
**2, rue de la Follie**
**B-7190 Ecaussines (BE)**

(72) Inventeur : **LEGROS, Franz**
**8, rue de Plagniau**
**B-133o Rixensart (BE)**
Inventeur : **LEROY, Claude-Pascal**
**2, rue de la Follie**
**B-7190 Ecaussines (BE)**

(74) Mandataire : **Van Malderen, Michel**
**p.a. Office van Malderen avenue J.S. Bach 22/43**
**B-1080 Bruxelles (BE)**

Jouve, 18, rue Saint-Denis, 75001 PARIS

## Description

La présente invention concerne une composition pharmaceutique contenant un composé actif hydrophile traité par un acide organique, de façon à obtenir la formation d'un complexe entre les deux molécules, et encapsulé dans un liposome.

De nombreuses substances présentent des propriétés pharmacologiques, mais ne sont pas utilisées à leur efficacité optimale parce qu'elles ne possèdent pas toutes les qualités d'un bon composé pharmaceutique. Remédier à l'action insuffisamment puissante de ces médicaments par une augmentation des doses conduirait à une toxicité certaine. Ainsi, si pour pallier une action trop faible ou de durée trop courte, on injecte une plus forte dose de composé actif, celui-ci risquerait de produire une forte toxicité ou des effets secondaires (allergiques, digestifs, rénaux, nerveux, hématologiques, etc). Spécifiquement, la néphrotoxicité, l'ototoxicité et la neurotoxicité de la gentamycine et des autres antibiotiques aminoglucosidiques ont été abondamment décrites.

De plus, des publications sur l'encapsulation des antibiotiques tels que la gentamycine (Morgan, J.R. et Williams K.E., l980, Anti microb. Agents Chemother.l7: 544-548; Barza, M., Baum, J. et Szoka, F., 1984, Invest. Ophtalmol. Vis. Sci. 25:486-490) laissent apparaître que les auteurs n'ont obtenu que des taux d'encapsulation compris entre 5% et 25%.

D'autres publications (WO-A1-88/04573, The Liposome Company; WO-A1-87/00043, The Liposome Company; Schreier, H. et coll., 1987, J. Controlled Release 5: 187-192) mentionnent dans des techniques d'encapsulation d'antibiotiques, uniquement l'utilisation de molécules inorganiques.

Une utilisation d'acides organiques pour la préparation de gel constitué de liposomes déshydratés est connue par un brevet européen (EP-A1-0211647), mais sans conjugaison avec des molécules pharmaceutiques et à des pH beaucoup plus élevés que ceux des molécules encapsulées. L'acide organiques n'est utilisé que pour la réhydratation des liposomes.

Le brevet WO-A-87/00043 concerne essentiellement des liposomes multilamellaires pouvant renfermer diverses substances actives, entre autres, des drogues hydrophiles telles que la gentamycine: voir page 15, lignes 16 et suivantes de ce document.

L'article, Journal of Controlled Release 5 (1987), pp. 187-192, est relatif à des liposomes renfermant du sulfate de gentamycine. L'acide sulfurique n'est pas un acide organique.

La présente invention vise à fournir un produit qui, après encapsulation, présente la propriété intéressante d'être auto-régulant, en ce sens qu'il libère le composé actif en raison inverse de la concentration de ce composé dans le milieu extérieur. Ceci présente l'avantage de garantir une action prolongée des antibiotiques administrés, tout en évitant l'apparition de doses toxiques ou de doses inefficaces.

Ces propriétés sont particulièrement intéressantes pour certaines applications, notamment ophtalmologiques, qui seront décrites ci-après et pour lesquelles la présence d'un composé actif en trop forte concentration peut être la cause de lésions irréversibles. A titre complémentaire, le produit de l'invention présente également des taux d'encapsulation élevés, pouvant atteindre 50%.

Afin d'atteindre les buts ainsi visés, l'invention propose une composition pharmaceutique contenant un composé actif hydrophile traité par un acide organique, de façon à assurer la conjugaison des deux molécules, et encapsulé dans un liposome. Cette composition est caractérisable par un volume intraliposomial maximal lorsque le composé actif hydrophile est lié au(x) groupement(s) carboxylique(s) de l'acide. Parmi les composés actifs hydrophiles, on accorde la préférence aux antibiotiques aminoglucosidiques.

On entend par antibiotique aminoglucosidique une molécule comportant des résidus glucidiques qui comportent des groupes $NH_2$ en chaîne latérale. Le terme couvre donc d'autres molécules que celles répondant à la définition pharmacologique classique d'antibiotiques dits aminoglucosidiques.

En particulier, la gentamycine est traitée par un acide organique à un pH de l'ordre de 4,5.

Parmi les acides organiques, on accorde la préférence aux acides aminés, en particulier l'acide glutamique, ou à des acides multicarboxyliques tels que l'acide succinique ou l'acide citrique.

Cependant, des acides simples tels que l'acide acétique ou l'acide formique peuvent également convenir.

Préférentiellement, les liposomes mis en oeuvre sont du type dit "multilamellaires" (MLV) avec cholestérol.

On accorde la préférence, selon l'invention, généralement à des liposomes multilamellaires avec cholestérol présentant des rapports molaires Egg Pc/Chol compris entre 4/1 et 4/6, ce dernier rapport ou des valeurs proches de celui-ci étant tout particulièrement préférés.

L'invention propose également un procédé d'obtention du produit en procédant à une encapsulation directe du composé actif hydrophile, c'est-à-dire que le composé actif hydrophile est mis en contact avec l'acide et est encapsulé avec formation d'un liposome.

Du fait qu'on observe une libération du principe actif encapsulé en raison inverse de la présence de celui-ci dans le milieu extérieur, le produit de l'invention présente un intérêt considérable. Les applications peuvent être locales ou systémiques et le liposome peut être choisi de façon à obtenir des effets complémentaires tels

2

qu'un ciblage en fonction du tropisme du liposome. Des formes d'application chargées du liposome peuvent être utilisées notamment pour provoquer un dépôt préférentiel sur une membrane ou un tissu.

La présentation préférentielle du produit de l'invention est sous forme d'une suspension liposomiale à un pH compris entre 4,5 et 7,5.

Une telle préparation, après un éventuel réajustement au pH physiologique, peut être appliquée par voie parentérale ou en aérosol et une application per os peut être envisagée, alors que des composés tels que la gentamycine ne sont normalement pas résorbés.

Avantageusement, les produits de l'invention sont également utilisés pour la préparation d'un médicament antibiotique a usage local, notamment ophtalmologique.

Le produit de l'invention est caractérisé par la conjugaison d'un composé actif hydrophile et de(s) groupement(s) des acides organiques, suivie de l'encapsulation dans un liposome.

Le produit de l'invention sera décrit plus en détail en référence aux essais et à des exemples pratiques de mise en oeuvre de son procédé d'obtention.

Différents paramètres ont été successivement étudiés pour démontrer l'effet de ceux-ci sur la technique proposée par l'invention.

### Conjugaison de la gentamycine et de l'acide glutamique à pH 4,5.

La liaison spécifique entre le composé actif hydrophile et les groupements carboxyliques de l'acide organique est caractérisée par un titrage acido-basique de la conjugaison du composé actif hydrophile et de l'acide organique.

L'acide glutamique 0.998M a été titré avec du KOH 0.l0lM et de l'HCl 0.999M (Fig la). La gentamycine sulfatée 0.998M a été titrée selon le même procédé (Fig. lb). Les deux molécules ont ensuite été mélangées mole à mole et titrées. La figure lc montre la disparition d'un des groupements carboxyliques de l'acide glutamique à un pH de l'ordre de 4.5.

### Encapsulation liposomiale de gentamycine dans l'acide glutamique.

L'encapsulation de gentamycine a été mesurée une heure après la formation des liposomes.

### Condition de travail.

- Composition liposomiale: phosphatidylcholine de jaune d'oeuf (Egg Pc) et cholestérol (Chol).
- Rapports molaires entre les constituants: 4/3 et 4/6.
- Masse totale des constituants lipidiques: 60 mg.
- Quantités de gentamycine: l, l0 et 20 mg.
- Volumes d'encapsulation: l, 0.5 et 0.25 ml.
- pH d'encapsulation: 4,5 et 7,5.
- Tampon d'encapsulation: acide glutamique/KOH (Osmolalité: 285 mOsm/kg $H_2O$).

### Méthode d'encapsulation liposomiale.

- Formation d'un film sec d'Egg Pc et de cholestérol;
- Addition du mélange acide glutamique-gentamycine;
- Mise en suspension des liposomes par vortexation;
- Incubation pendant l heure à température ambiante;
- Centrifugation à 5000 rpm pendant 10 minutes;
- 3 lavages du culot liposomial par une solution tamponnée d'acide glutamique au même pH que celui utilisé pour l'encapsulation.

Les résultats de divers essais sont regroupés dans le Tableau 1.

## Tableau 1.Efficience d'encapsulation (%) de gentamycine mélangée à l'acide glutamique en fonction du pH, de la composition liposomiale et du volume d'encapsulation.

| pH | mg genta | rapport molaire | volume ml | %d'encap ± dev. st. | N |
|----|----------|-----------------|-----------|---------------------|---|
| 4,5 | 1 | 4/3 | 1 | 10,5±1 | 24 |
| 4,5 | 1 | 4/3 | 0,5 | 18,2±0,3 | 3 |
| 4,5 | 1 | 4/3 | 0,25 | 21,6 | 1 |
| 4,5 | 1 | 4/3 | 1 | 7,8±0,9 | 3 |
| 7,5 | 1 | 4/3 | 1 | 10,8±1,5 | 26 |
| 4,5 | 1 | 4/6 | 1 | 33,5±3 | 10 |
| 4,5 | 1 | 4/6 | 0,5 | 37,5±5 | 3 |
| 4,5 | 1 | 4/6 | 0,25 | 46,1 | 1 |
| 4,5 | 10 | 4/6 | 1 | 35,4±3,5 | 4 |
| 4,5 | 20 | 4/6 | 1 | 35±3,5 | 8 |
| 7,5 | 1 | 4/6 | 1 | 33,5±3 | 6 |

L'efficience d'encapsulation varie de 8 à 46%. L'encapsulation optimale a été obtenue avec des liposomes de composition 4/6, à pH 4,5 et dans un volume de 0,25 ml.

Influence du pH de l'acide glutamique sur l'efficience d'encapsulation.

Conditions expérimentales:
- Concentration lipidique: 60 mg/ml.
- Concentration de gentamycine: l mg/ml.
- pH de l'acide glutamique: 3; 4,5; 7,5.
- Temps d'incubation: l heure.

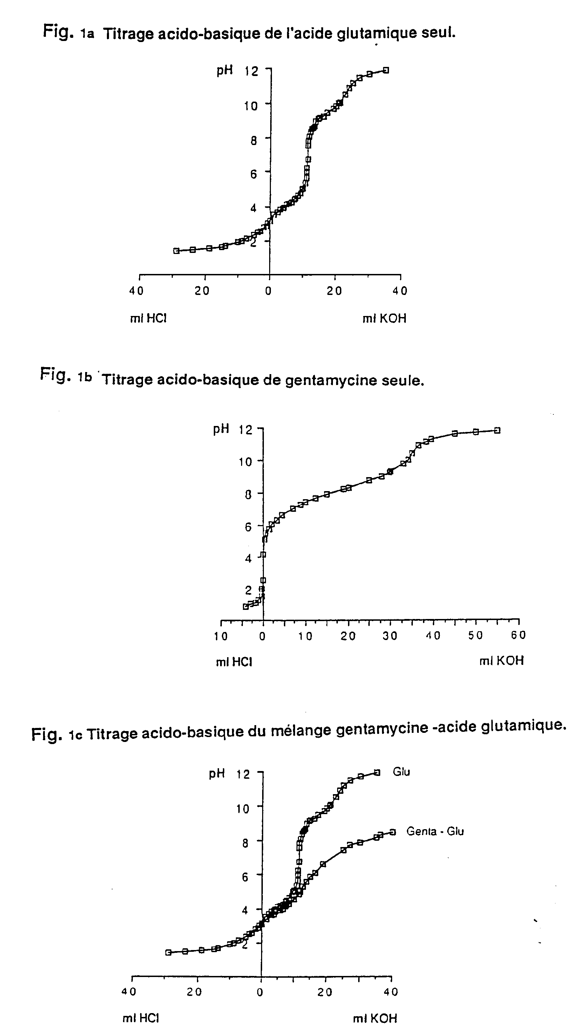

L'efficience d'encapsulation de la gentamycine croît de pH 3 à 4,5, où elle atteint son maximum. A pH 7,5, l'efficience est légèrement inférieure à celle mesurée à pH 4,5 (Voir Figure 2a).

Influence du pH de l'acide glutamique sur le volume liposomial.

Le volume liposomial a été mesuré par l'encapsulation d'inuline marquée par le $^{14}$C . Les conditions expérimentales sont les suivantes:
- Concentration de lipides: 60 mg/ml.
- Concentration de gentamycine: 20 mg/ml.
- pH de l'acide glutamique: 3; 4,5; 7,5.
- pH du tampon Tris-HCl: 7,5.
- Temps d'incubation: l heure.

Le Tableau 2 montre qu'à pH 7,5, la présence d'acide glutamique n'augmente pas le volume intraliposomial par rapport au Tris. Dans le tampon Tris-HCl pH 7,5, l'addition de gentamycine modifie le volume des liposomes. Par contre, l'addition de gentamycine à l'acide glutamique à pH 7,5 agrandit plus fortement la taille des liposomes.

## Tableau 2.Variation du volume intraliposomial en fonction du pH et de la composition du milieu d'encapsulation.

| pH | liposomes avec gentamycine | | liposomes sans gentamycine | |
|----|----------|---------|----------|---------|
|    | glu / KOH | Tris-HCl | glu / KOH | Tris-HCl |
| 4,5 | 176±5 µl | | 90±19 µl | |
| 7,5 | 73,3±1µl | 67±0,6 µl | 53± 3 µl | 54±5 µl |

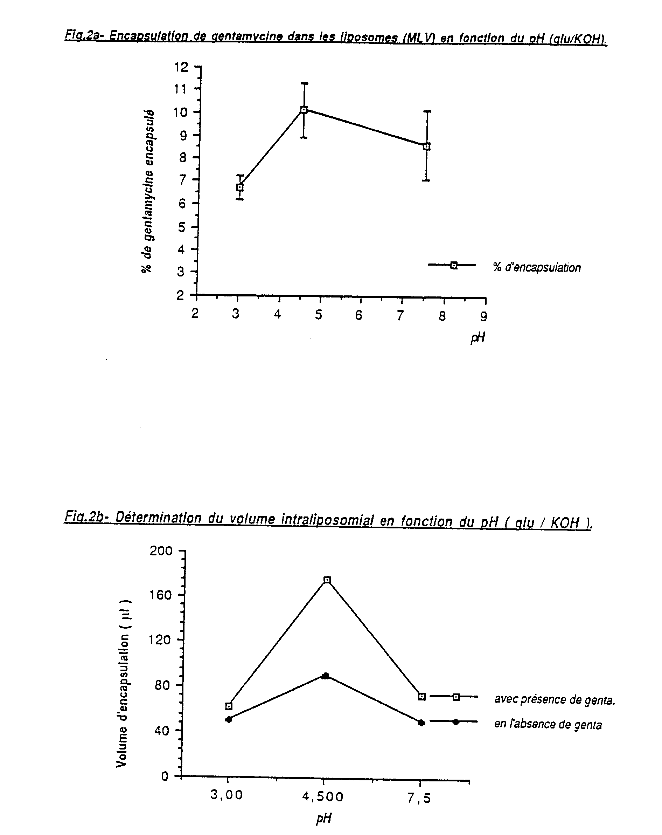

L'addition de gentamycire à l'acide glutamique utilisé comme tampon augmente le volume liposomial aux trois pH. Cette augmentation est toutefois nettement plus marquée à pH 4,5 où la présence de gentamycine double le volume liposomial (Tableau 2 et Fig.2b).

Encapsulation liposomiale dans d'autre acides organiques.

L'encapsulation de gentamycine à pH 4,5 a été effectuée dans de l'acide succinique, de l'acide acétique et de l'acide formique. Les efficiences d'encapsulation étaient de l'ordre de celles obtenues dans l'acide glutamique aux mêmes conditions expérimentales.

Spécifiquement, des encapsulations ont été effectuées dans un tampon citrate/acétate utilisé pour l' administration ophtalmologique.

# Tableau 3. Efficience d'encapsulation (%) de gentamycine mélangée à un tampon citrate/acétate en fonction du pH et de la composition liposomiale (60 mg de lipides).

| pH | conc. genta mg/ml | rapport molaire | %d'encap ± dev. st. | N |
|----|----|----|----|----|
| 4,5 | 1 | 4/3 | 15,9±6,3 | 3 |
| 7,5 | 1 | 4/3 | 12,5±2,1 | 3 |
| 4,5 | 1 | 4/6 | 34±2,8 | 3 |
| 7,5 | 1 | 4/6 | 35±4,1 | 3 |

A nouveau, l'encapsulation optimale (35%) est obtenue lorsque la gentamycine mélangée au tampon, à pH 4,5 ou 7,5, est encapsulée dans des structures liposomiales dont le rapport molaire Egg Pc/Chol. est de 4/6.

Libération de la gentamycine encapsulée (release).

Dans le but de déterminer le temps de sortie des liposomes et la dose optimale de gentamycine libérée après encapsulation à pH 3, 4,5 et 7,5, on a étudié sa cinétique de libération dans deux milieux physiologiques:
- Humeur vitrée de boeuf à pH 7,2, 300 mOsmol/kg $H_2O$,
- Plasma de lapin à pH 7,2, 287 mOsmol/kg $H_2O$.
La libération de gentamycine s'est effectuée à 37°C.
Conditions expérimentales:
- Concentration de lipides: 60 mg/ml.
- Concentration de gentamycine: 1 mg/ml.
- Rapport molaire Egg Pc/Chol: 4/3.
- pH d'encapsulation dans l'acide glutamique: 3; 4,5; 7,5.
Après formation des liposomes et lavage, le culot liposomial est suspendu dans l ml d'humeur vitrée ou de plasma. La gentamycine restant dans les liposomes est mesurée à différents temps.

Sortie dans le plasma de lapin (Figure 3a).

La sortie de gentamycine des liposomes formés à pH 4,5 dans l'acide glutamique est progressive. Lorsque la gentamycine libérée dans le plasma atteint une valeur critique, la drogue cesse de sortir des liposomes. Si le plasma est renouvelé, la sortie reprend (voir la Figure 3a annexée, où W représente le remplacement du plasma chargé en gentamycine par du plasma frais)
Après plusieurs lavages de ce type, les liposomes peuvent être complètement vidés de la drogue. Ceci signifie qu'une fois introduits dans les liquides biologiques, les liposomes peuvent libérer progressivement, d'une manière rétrocontrôlée par la concentration de gentamycine extraliposomiale, la totalité de la drogue qui y était encapsulée.

Sortie dans l'humeur vitrée.

Lorsque la gentamycine a été encapsulée dans du tampon glutamique à pH 4,5 ou 7,5, la sortie de drogue dans l'humeur vitrée se passe de la même façon que dans le plasma (Figure 3b).
- Sortie progressive;
- Saturation de l'humeur vitrée;
- Reprise de sortie après renouvellement de l'humeur vitrée.

Le pourcentage de gentamycine libérée avant saturation de l'humeur vitrée est plus important à pH 7,5 qu'à pH 4,5. Le choix du pH d'encapsulation dans l'acide glutamique permet donc d'offrir diverses formes pharmaceutiques possédant des propriétés de sortie de la drogue qui peuvent correspondre à divers besoins cliniques.

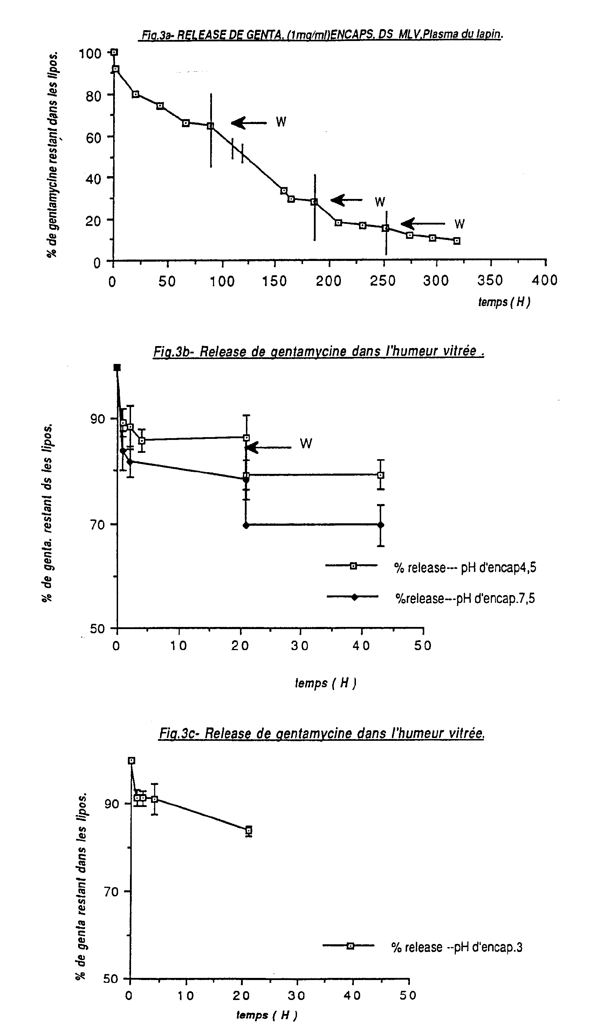

Par contre, la sortie de gentamycine encapsulée dans l'acide glutamique à pH 3 ne présente pas cette forme de rétrocontrôle par la concentration de la drogue dans le milieu physiologique. La sortie de la drogue s'opère de façon continue (Figure 3 c).

Libération de gentamycine dans l'humeur vitrée de lapin in vivo.

Conditions expérimentales:
- Liposomes: 20 mg/ml de gentamycine et 20 mg/ml de lipides (Egg Pc/Chol, rapport molaire 4/6); temps d'encapsulation: I heure.
- Injection dans l'humeur vitrée de lapins anesthésiés de 0,I ml de culot liposomial contenant I milligramme de gentamycine.
- Sacrifice des lapins après 24 et 48 heures et prélèvement de l'humeur vitrée.
- Centrifugation de l'humeur (5000 rpm, 20 minutes), séparation du culot liposomial et de l'humeur, mesure de la quantité de gentamycine dans les deux fractions.
- Les résultats sont exprimés comme le rapport de la quantité de gentamycine libérée dans l'humeur vitré/ la quantité restant dans les liposomes (Tableau 4).

## Tableau 4. Libération de gentamycine dans l'humeur vitrée in vivo.

| lapins | temps | rapport surnageant/culot |
| --- | --- | --- |
| n°1 | 24 H | 7,79% |
| n°2 | 24 H | 6,26% |
| n°3 | 48 H | 6,83% |
| n°4 | 48 H | 5,09% |

De 24 à 48 heures après l'injection, le rapport reste constant. Ceci indique l'existence d'une libération lente et constante de la gentamycine dans les liquides biologiques in vivo.

## Revendications

1. Composition pharmaceutique contenant un antibiotique hydrophile aminoglucosidique sous forme d'un conjugué avec un acide organique, encapsulé dans un liposome.

2. Composition pharmaceutique selon la revendication 1, caractérisée en ce que l'antibiotique aminoglucosidique est la gentamycine sulfatée qui est traitée par un acide organique à un pH de l'ordre de 4,5 à 7,5, de préférence de l'ordre de 4,5.

3. Composition pharmaceutique selon les revendications 1 ou 2, caractérisée en ce que les acides organiques utilisés sont choisis parmi les acides aminés, en particulier l'acide glutamique, ou les acides multicarboxyliques tels que l'acide succinique ou l'acide citrique ou les acides simples tels que l'acide acétique et l'acide formique.

4. Composition pharmaceutique selon l'une quelconque des revendications précédentes, caractérisée en ce que les liposomes mis en oeuvre sont du type "multilamellaires" (MLV), de préférence avec du cholestérol.

5. Composition pharmaceutique selon l'une quelconque des revendications précédentes, caractérisée en ce que les liposomes multilamellaires utilisés avec cholestérol présentent des rapports Egg Pc/Chol (phosphatidylcholine de jaune d'oeuf/cholestérol) compris entre 4/1 et 4/6, de préférence de l'ordre de 4/6.

6. Procédé pour la préparation d'une composition pharmaceutique selon l'une quelconque des revendications précédentes, caractérisé en ce que le composé actif hydrophile est mis en contact avec l'acide et est ensuite encapsulé avec formation d'un liposome.

7. Utilisation des produits selon l'une quelconque des revendications précédentes pour la préparation de médicaments antibiotiques destinés à être appliqués, après un éventuel réajustement au pH physiologique, par voie parentérale, entérale ou en aérosol.

8. Utilisation des produits selon l'une quelconque des revendications précédentes pour la préparation d'un médicament antibiotique à usage local, en particulier ophtalmologique.


**Patentansprüche**

1. Pharmazeutische Zusammensetzung, die ein hydrophiles Aminoglukosid-Antibiotikum in form einer in ein Liposom eingekapselten, konjugierten Verbindung mit einer organischen Säure enthält.

2. Pharmazeutische Zusammensetzung gemäß Anspruch 1, dadurch gekennzeichnet, daß das Aminoglukosid-Antibiotikum das sulfatierte Gentamycin ist, das mit einer organischen Säure bei einem pH von ungefähr 4,5 bis 7,5, vorzugsweise 4,5, behandelt wird.

3. Pharmazeutische Zusammensetzung gemäß den Ansprüchen 1 oder 2, dadurch gekennzeichnet, daß die verwendeten organischen Säuren unter den Aminosäuren, wie insbesondere die Glutaminsäure, oder den Multicarbonsäuren, wie die Succinsäure oder die Zitronensäure, oder den einfachen Säuren, wie die Essigsäure und die Ameisensäure, gewählt werden.

4. Pharmazeutische Zusammensetzung gemäß irgendeinem der vorhergehenden Ansprüche, dadurch gekennzeichnet, daß die verwendeten Liposomen vom "multilamellaren" Typ (MLV) sind, vorzugsweise mit Cholesterin.

5. Pharmazeutische Zusammensetzung gemäß irgendeinem der vorhergehenden Ansprüche, dadurch gekennzeichnet, daß die mit Cholesterin verwendeten, multilamellaren Liposomen Verhältnisse Egg Pc/Chol (Eigelb-Phosphatidylcholin/Cholesterin) zwischen 4/1 und 4/6, vorzugsweise von ungefähr 4/6 aufweisen.

6. Verfahren zur Herstellung einer pharmazeutischen Zusammensetzung gemäß irgendeinem der vorhergehenden Ansprüche, dadurch gekennzeichnet, daß die hydrophile wirksame Verbindung mit der Säure in Kontakt gebracht wird, und danach unter Bildung eines Liposoms eingekapselt wird.

7. Verwendung der Produkte gemäß irgendeinem der vorhergehenden Ansprüche zur Herstellung von antibiotischen Medikamenten, die dazu bestimmt sind, nach einer eventuellen Anpassung an den physiologischen pH, auf parenteralem oder enteralem Wege, oder als Aerosol angewandt zu werden.

8. Verwendung der Produkte gemäß irgendeinem der vorhergehenden Ansprüche zur Herstellung eines antibiotischen Medikaments für örtliche, insbesondere ophthalmologische Anwendung.

**Claims**

1. Pharmaceutical composition containing an hydrophilic aminoglucoside antibiotic under the form of a conjugate with an organic acid, encapsulated in a liposome.

2. Pharmaceutical composition according to claim 1, characterized in that the aminoglucoside antibiotic is sulfated gentamycin which is treated with an organic acid at a pH of the order of 4.5 to 7.5, preferably of the order of 4.5.

3. Pharmaceutical composition according to claims 1 or 2, characterized in that the organic acids used are chosen from among the amino acids, in particular glutamic acid, or the polycarboxylic acids such as succinic acid or citric acid, or the simple acids such as acetic acid and formic acid.

4. Pharmaceutical composition according to any of the preceding claims, characterized in that the liposomes employed are of the "multilamellar" (MLV) type, preferably with cholesterol.

5. Pharmaceutical composition according to any of the preceding claims, characterized in that the multilamellar liposomes with cholesterol are used which exhibit Egg phosphatidylcholine (PC)/cholesterol (Ch) ratios of between 4/1 and 4/6, preferably of the order of 4/6.

6. Process for the preparation of a composition according to any of the preceding claims, characterized in that the hydrophilic active compound is brought into contact with the acid and is thereafter encapsulated, with formation of a liposome.

7. Use of the products according to any of the preceding claims, for the preparation of medicaments intended to be applied parenterally, enterally or as an aerosol, if necessary after readjustment to the physiological pH.

8. Use of the products according to any of the preceding claims for the preparation of an antibiotic medicament for local application, in particular ophthalmological application.

**Fig. 1a** Titrage acido-basique de l'acide glutamique seul.

pH

ml HCl          ml KOH

**Fig. 1b** Titrage acido-basique de gentamycine seule.

pH

ml HCl          ml KOH

**Fig. 1c** Titrage acido-basique du mélange gentamycine -acide glutamique.

pH          Glu

Genta - Glu

ml HCl          ml KOH

10

_Fig.2a- Encapsulation de gentamycine dans les liposomes (MLV) en fonction du pH (glu/KOH)._

_Fig.2b- Détermination du volume intraliposomial en fonction du pH ( glu / KOH )._

Fig.3a- RELEASE DE GENTA. (1mg/ml)ENCAPS. DS  MLV,Plasma du lapin.

Fig.3b- Release de gentamycine dans l'humeur vitrée .

Fig.3c- Release de gentamycine dans l'humeur vitrée.